(19) Europäisches Patentamt
European Patent Office
Office européen des brevets

(11) **EP 2 361 583 B1**

(12) **EUROPEAN PATENT SPECIFICATION**

(45) Date of publication and mention
of the grant of the patent:
**20.04.2016 Bulletin 2016/16**

(51) Int Cl.:
***A61B 18/18*** *(2006.01)*     ***A61B 18/00*** *(2006.01)*
***A61B 17/00*** *(2006.01)*

(21) Application number: **11001596.3**

(22) Date of filing: **25.02.2011**

(54) **System for monitoring ablation size**

System zur Überwachung der Ablationsgröße

Système de surveillance de la taille d'ablation

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **25.02.2010 US 712864**

(43) Date of publication of application:
**31.08.2011 Bulletin 2011/35**

(73) Proprietor: **Covidien LP
Mansfield, MA 02048 (US)**

(72) Inventor: **Brannan, Joseph D.
Erie, CO 80516 (US)**

(74) Representative: **Soames, Candida Jane et al
Maschio & Soames IP Limited
20 Carlton Crescent
Southampton, SO15 2ET (GB)**

(56) References cited:
**US-A- 6 132 425     US-B1- 6 500 175**

## Description

## BACKGROUND

*Technical Field*

The present disclosure relates to systems and methods that may be used in tissue ablation procedures. More particularly, the present disclosure relates to systems and methods for monitoring ablation size during tissue ablation procedures in real-time.

*Background of Related Art*

In the treatment of diseases such as cancer, certain types of cancer cells have been found to denature at elevated temperatures (which are slightly lower than temperatures normally injurious to healthy cells). These types of treatments, known generally as hyperthermia therapy, typically utilize electromagnetic radiation to heat diseased cells to temperatures above 41° C while maintaining adjacent healthy cells at lower temperatures where irreversible cell destruction will not occur. Procedures utilizing electromagnetic radiation to heat tissue may include ablation of the tissue.

Microwave ablation procedures, e.g., such as those performed for menorrhagia, are typically done to ablate the targeted tissue to denature or kill the tissue. Many procedures and types of devices utilizing electromagnetic radiation therapy are known in the art. Such microwave therapy is typically used in the treatment of tissue and organs such as the prostate, heart, and liver.

One non-invasive procedure generally involves the treatment of tissue (e.g., a tumor) underlying the skin via the use of microwave energy. The microwave energy is able to non-invasively penetrate the skin to reach the underlying tissue. However, this non-invasive procedure may result in the unwanted heating of healthy tissue. Thus, the non-invasive use of microwave energy requires a great deal of control.

Currently, there are several types of systems and methods for monitoring ablation zone size. In certain instances, one or more types of sensors (or other suitable devices) are operably associated with the microwave ablation device. For example, in a microwave ablation device that includes a monopole antenna configuration, an elongated microwave conductor may be in operative communication with a sensor exposed at an end of the microwave conductor. This type of sensor is sometimes surrounded by a dielectric sleeve.

Typically, the foregoing types of sensor(s) is configured to function (e.g., provide feedback to a controller for controlling the power output of a power source) when the microwave ablation device is inactive, i.e., not radiating. That is, the foregoing sensors do not function in real-time. Typically, the power source is powered off or pulsed off when the sensors are providing feedback (e.g., tissue temperature) to the controller and/or other device(s) configured to control the power source.

US 6,500,175 B1 discloses an antenna ablation apparatus for cell necrosis. The apparatus comprises an electrode for radiating a tissue. External floating probes comprising temperature sensors are provided for determining the extension of the ablation. Some of the probes can slide within lumens of the electrode and be positioned in an adapted region of the tissue.

US 6,132,425 A discloses a cell necrosis apparatus specifically meant for operating a volumetric cell necrosis. For this aim, the apparatus comprises a plurality of electrodes for providing RF energy and which are meant for spatially occupy the space for obtaining a conic ablation volume. These electrodes are slidable through an insulation sleeve which surrounds an introducer. Sensors for measuring temperature or impedance can be provided in the electrodes in particular at the distal end of each electrode and at the introducer distal end.

## SUMMARY

The invention is defined in claim 1.

The present disclosure provides a system for monitoring ablation size in real-time. The system includes a power source including a microprocessor for executing at least one control algorithm. A microwave antenna is configured to deliver microwave energy from the power source to tissue to form an ablation zone. A series of thermal sensors is operably disposed adjacent a radiating section of the microwave antenna and extends proximally therefrom. The thermal sensors corresponding to a radius of the ablation zone, wherein each thermal sensor generates a voltage when a predetermined threshold tissue temperature is reached corresponding to the radius of the ablation zone.

The present disclosure provides a microwave antenna adapted to connect to a power source configured for performing an ablation procedure. The microwave antenna includes a radiating section configured to deliver microwave energy from the power source to tissue to form an ablation zone. The microwave antenna includes a series of thermal sensors operably disposed adjacent the radiating section of the microwave antenna and extending proximally therefrom. The thermal sensors corresponding to a radius of the ablation zone, wherein each thermal sensor generates a voltage when a predetermined threshold tissue temperature is reached corresponding to the radius of the ablation zone.

The present disclosure also provides a method for monitoring temperature of tissue undergoing , which is however not part of the invention. The method includes the initial step of transmitting microwave energy from a power source to a microwave antenna to form a tissue ablation zone. A step of the method includes monitoring the proximal propagation of tissue temperature along the microwave antenna as the tissue ablation zone forms. Triggering a detection signal when a predetermined tissue temperature is reached at specific points along the microwave antenna is another step of the method. The method includes adjusting the amount of microwave energy from the power source to the microwave antenna.

## BRIEF DESCRIPTION OF THE DRAWINGS

The above and other aspects, features, and advantages of the present disclosure will become more apparent in light of the following detailed description when taken in conjunction with the accompanying drawings in which:

FIG. 1A is a perspective view of a system for monitoring ablation size according to an embodiment of the present disclosure;
FIG. 1B is a perspective view of a system for monitoring ablation size according to another embodiment of the present disclosure;
FIG. 2A is partial side view of a distal tip of a microwave antenna depicted in FIG. 1A;
FIG. 2B is partial side view illustrating internal components associated with a distal tip of a microwave antenna according to an alternate embodiment of the present disclosure;
FIG. 3A is a schematic, plan view of the tip of a microwave antenna depicted in FIG. 2A illustrating radial ablation zones having a spherical configuration;
FIG. 3B is a schematic, plan view of the tip of a microwave antenna depicted in FIG. 2A illustrating radial ablation zones having an ellipsoidal configuration;
FIG. 4 is a functional block diagram of a power source for use with the system depicted in FIG. 1A; and
FIG. 5 is a flow chart illustrating a method for monitoring temperature of tissue undergoing ablation in accordance with the present disclosure.

## DETAILED DESCRIPTION

Embodiments of the presently disclosed system and method are described in detail with reference to the drawing figures wherein like reference numerals identify similar or identical elements. As used herein and as is traditional, the term "distal" refers to the portion which is furthest from the user and the term "proximal" refers to the portion that is closest to the user. In addition, terms such as "above", "below", "forward", "reaward", etc. refer to the orientation of the figures or the direction of components and are simply used for convenience of description.

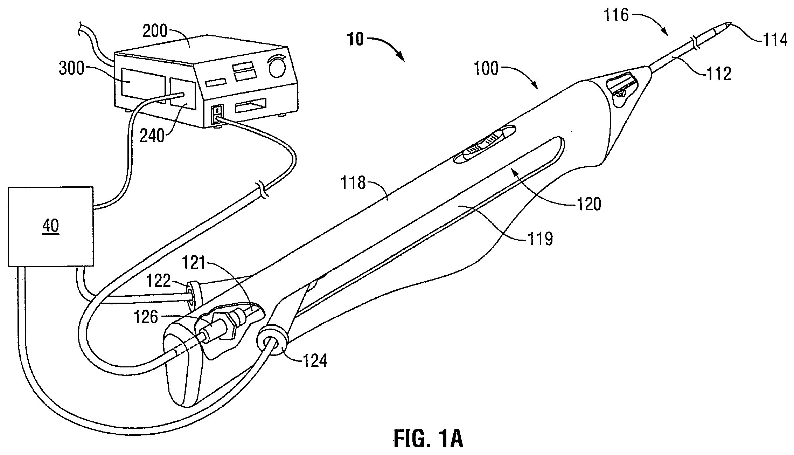

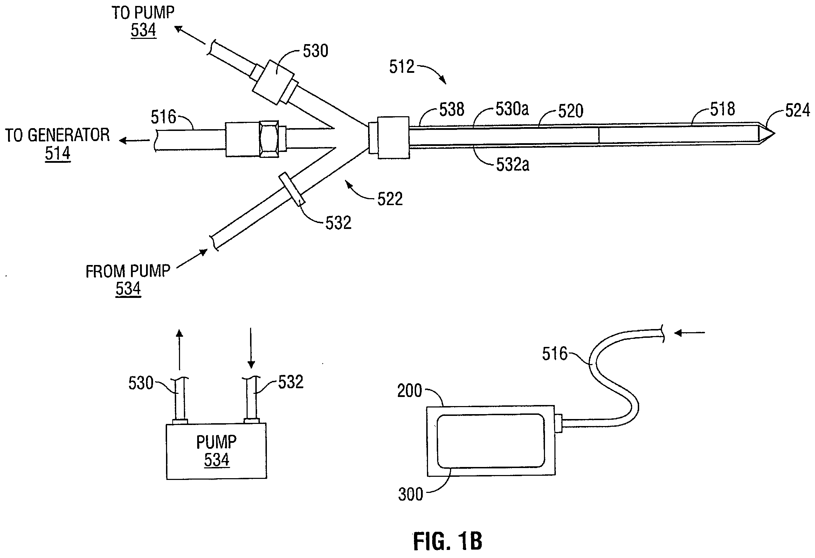

Referring now to FIG. 1A, a system for monitoring ablation size in accordance with an embodiment of the present disclosure is designated 10. The system 10 includes a microwave antenna 100 that is adapted to connect to an electrosurgical power source, e.g., an RF and/or microwave (MW) generator 200 that includes or is in operative communication with one or more controllers 300 and, in some instances, a fluid supply pump 40.

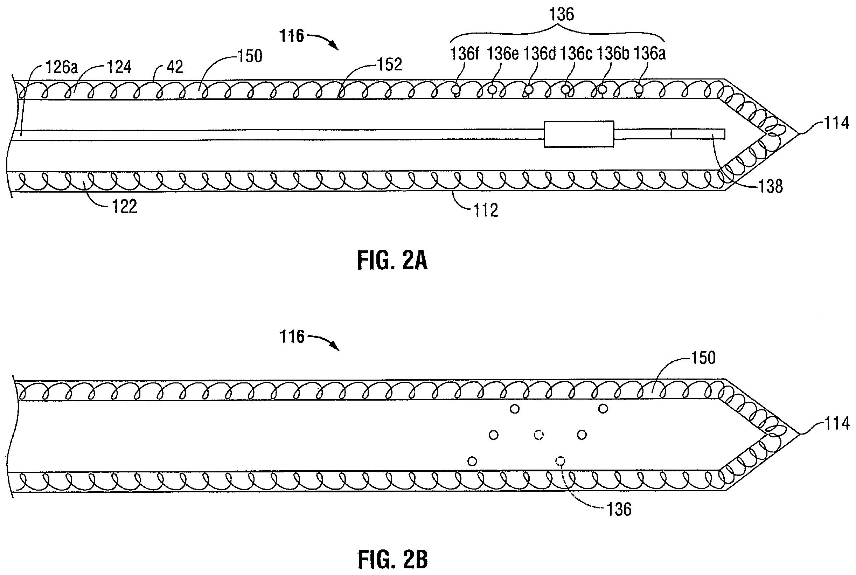

Briefly, microwave antenna 100 includes an introducer 116 having an elongated shaft 112 and a radiating or conductive section or tip 114 operably disposed within elongated shaft 112, a cooling assembly 120 having a cooling sheath 121, a handle 118, a cooling fluid supply 122 and a cooling fluid return 124, and an electrosurgical energy connector 126. Connector 126 is configured to connect the microwave antenna 100 to the electrosurgical power source 200, e.g., a generator or source of radio frequency energy and/or microwave energy, and supplies electrosurgical energy to the distal portion of the microwave antenna 100. Conductive tip 114 and elongated shaft 112 are in electrical communication with connector 126 via an internal coaxial cable 126a that extends from the proximal end of the microwave antenna 100 and includes an inner conductor tip that is operatively coupled to a radiating section 138 operably disposed within the shaft 112 and adjacent the conductive or radiating tip 114 (see FIG. 2A, for example). As is common in the art, internal coaxial cable 126a includes a dielectric material and an outer conductor surrounding each of the inner conductor tip and dielectric material. A connection hub (not shown) disposed at a proximal end of the microwave antenna 100 operably couples connector 126 to internal coaxial cable 126a, and cooling fluid supply 122 and a cooling fluid return 124 to a cooling assembly 120. Radiating section 138 by way of conductive tip 114 (or in certain instances without conductive tip 114) is configured to deliver radio frequency energy (in either a bipolar or monopolar mode) or microwave energy to a target tissue site. Elongated shaft 112 and conductive tip 114 may be formed of suitable conductive material including, but not limited to copper, gold, silver or other conductive metals having similar conductivity values. Alternatively, elongated shaft 112 and/or conductive tip 114 may be constructed from stainless steel or may be plated with other materials, e.g., other conductive materials, such as gold or silver, to improve certain properties, e.g., to improve conductivity, decrease energy loss, etc. In an embodiment, the conductive tip may be deployable from the elongated shaft 112.

In an alternate embodiment, system 10 may be configured for use with a microwave antenna 512 illustrated in FIG. 1A. Briefly, microwave antenna 512 operably couples to generator 200 including a controller 300 via a flexible coaxial cable 516. In this instance, generator 200 is configured to provide microwave energy at an operational frequency from about 500 MHz to about 10 GHz. Microwave antenna 512 includes a radiating section or portion 518 that may be connected by a feedline or shaft 520 to coaxial cable 516 that extends from the proximal end of the microwave antenna 512 and includes an inner conductor operably disposed within the shaft 520 and adjacent radiating section 518 and/or a conductive or radiating tissue piercing tip 524. More specifically, the microwave antenna 512 is coupled to the cable 516 through a connection hub 522. The connection hub 522 also includes an outlet fluid port 530 (similar to that of cooling fluid return 124) and an inlet fluid port 532 (similar to that of cooling fluid supply 122) that are connected in fluid communication with a sheath 538. The sheath 538 encloses the radiating portion 518 and the shaft 520 allowing for coolant fluid from the ports 530 and 532 to be supplied to and circulated around the antenna assembly 512 via respective fluid lumens 530a and 532a. The ports 530 and 532 may also couple to supply pump 40. For a more detailed description of the microwave antenna 512 and operative components associated therewith, reference is made to commonly-owned U.S. Patent Application Serial No. 12/401,268 filed on March 10, 2009.

For the remainder of the disclosure the operative components associated with the system 10 are described with reference to microwave antenna 100.

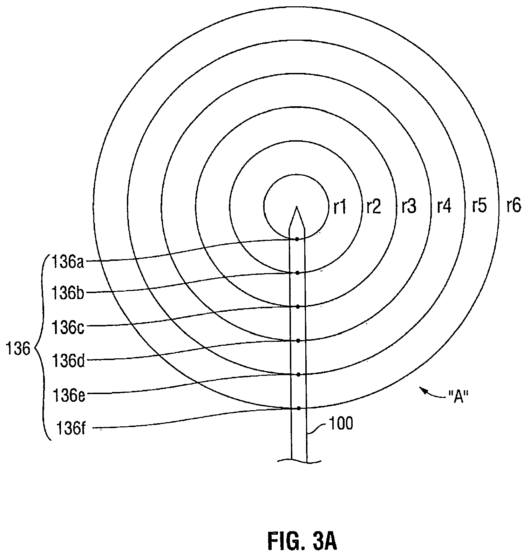

With reference now to FIG. 2A, the thermal sensors 136 are operably disposed along a length of the shaft 112 adjacent a radiating section 138 of the microwave antenna 100. In the embodiment illustrated in FIG. 2A, thermal sensor 136 includes a series of six thermal sensors labeled 136a-136f (collectively referred to as thermal sensors 136). As defined herein, a series of thermal sensors is meant to mean at least two thermal sensors. Thermal sensors 136 enable physical space sampling of the ablation site. Thermal sensors 136 may be operably positioned within an internal portion 150 of the shaft 112. More particularly, the thermal sensors 136 are operably disposed within an internal generally circumferential wall 152 that surrounds and/or defines the cooling fluid supply and fluid return lines 122 and 124, see FIG. 2A, for example. Thermal sensors 136 may be secured to the circumferential wall 152 via any suitable methods. In one particular embodiment, the thermal sensors 136 are secured to the circumferential wall via an epoxy adhesive. In an alternate embodiment, a plastic (or other suitable structure) provides mechanical fixation points for the thermal sensors 136. In this instance, the plastic structure (e.g., a circumferential or spiral plastic structure) may be operably disposed within the shaft 112 and couple to one or both of the fluid paths, e.g., supply fluid and return paths 122 and 124. Thermal sensors 136 may be disposed along the length of the shaft 112 in any suitable configuration. For example, in the embodiment illustrated in FIG. 2A, thermal sensors 136 are positioned along the shaft 110 in a linear manner forming a generally linear array along circumferential wall 152 of the shaft 112. Alternatively, the thermal sensors 136 may be positioned along circumferential wall 152 of the shaft 112 in a radially offset configuration forming a generally spherical or spiral array (FIG. 2B). Thermal sensors 136 may be any suitable thermal sensors 136 including but not limited to thermistors, thermocouples, fiber optic sensors, or other device suitable for the intended purpose for monitoring temperature. In the embodiment illustrated in FIGS. 2A and 3A, the thermal sensors 136 are six thermocouples 136a-136f. Thermal sensors 136 are in operative communication with a temperature module 332 and/or controller 300. More particularly, the thermal sensors 136 couple to the generator 200 and/or controller 300 via one or more leads, e.g., the internal cable 126a that extends from the proximal end of the microwave antenna 100 and connects to the port 126. In embodiments, the thermal sensors 136 may be in operative communication with the generator 200 and/or controller 300 by way of a wireless connection. Thermal sensors 136 are configured to provide comprehensive monitoring of an ablation zone "A" (FIG. 3A). The thermal sensors 136 are configured to generate an analog response (e.g., a voltage, resistance, current, etc.) when a predetermined threshold temperature is reached within the ablation zone "A." More particularly, an initial voltage potential is generated by each of the thermal sensors 136 during transmission of electrosurgical energy from the generator 200 to the microwave antenna 100. A predetermined threshold voltage potential is associated with each of thermal sensors 136 and corresponds to a predetermined threshold tissue temperature along a radius of an ablation zone (FIG. 3A). For example, thermal sensor 136a may be configured to generate an initial voltage potential of 1 volt and a predetermined threshold voltage of 2 volts that corresponds to a threshold tissue temperature of 60° Celsius that corresponds to an ablation zone "A" having a radius r1. The analog response, e.g., voltage, indicates to temperature module 332 that a predetermined threshold temperature is reached at the ablation zone (described in more detail below). More particularly, temperature module 332 monitors the thermal sensors 136, e.g., monitors voltage of the thermal sensors 136, and triggers a command signal in response to the thermal sensors 136 reaching a predetermined voltage such that the electrosurgical output power from the generator 200 may be adjusted. In an embodiment, an amplifier (not shown) may be utilized to amplify the voltage potential generated by the thermal sensors 136.

For a given microwave antenna 100, each of the thermal sensors 136 may be configured to generate an analog response, e.g., a predetermined voltage, when a predetermined threshold temperature, e.g., a threshold temperature approximately equal to 60° Celsius, has been met such that an ablation zone "A" having a radius r is created. The predetermined threshold temperature associated with a corresponding thermal sensor 136 and a corresponding radius r may be determined via any suitable methods. For example, predetermined threshold temperatures may be determined via known experimental data, model equations, or combination thereof. In one particular embodiment, one or more control algorithms for predicting tissue ablation size are employed by controller 300. More particularly, the concept of the integration of tissue temperature over time may be used to indicate tissue damage, e.g., death or necrosis. The control algorithm utilizes one or more model equations to calculate tissue damage that corresponds to tissue ablation zone size and temperature over time and at different energy levels. One suitable equation is the Arrhenius Equation, which may be represented by:

$$(1) \quad \Omega(t) = -\ln\left(\frac{c(t)}{c(0)}\right) = A \int_0^t e^{\left(\frac{-\Delta E}{RT}\right)} dt$$

where $\Omega$ represents damage sustained by tissue and c(t)/c(0) is the ration of the concentration of a component of interest at time equal to the original concentration, A is equal to the frequency factor, $E_a$ is activation energy, T is temperature (in absolute temperature), and R is the gas constant. Thus, for a given amount of tissue damage (which correlates to ablation zone having a radius r) a corresponding value of tissue temperature can be determined. The Arrhenius Equation is one of many equations that may be employed to calculate and predict tissue temperature over time and at different energy fields such that real-time monitoring of an ablation zone may be achieved.

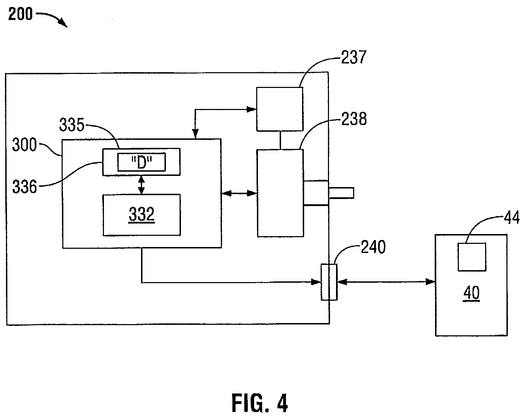

With reference to FIG. 4, a schematic block diagram of the generator 200 is illustrated. The generator 200 includes a controller 300 including one or more modules (e.g., a temperature module 332), a power supply 237, a microwave output stage 238. In this instance, generator 200 is described with respect to the delivery of microwave energy. The power supply 237 provides DC power to the microwave output stage 238 which then converts the DC power into microwave energy and delivers the microwave energy to the radiating section 138 of the microwave antenna 100 (see FIG. 2A). The controller 300 may include analog and/or logic circuitry for processing sensed analog responses generated by the thermal sensors 136 and determining the control signals that are sent to the generator 200 and/or supply pump 40 via the microprocessor 335. The controller 300 accepts one or more measurements signals indicative of a temperature associated with tissue adjacent an ablation zone and/or the microwave antenna 100, namely, the signals generated as a result of the analog response produced by temperature sensors 136. One or more modules e.g., temperature module 332, of the controller 300 monitors and/or analyzes the analog response, e.g., a generated voltage, produced by the temperature sensors 136 and determines if a threshold temperature has been met. If the threshold temperature has been met, then the temperature module, a microprocessor 335 and/or the controller instructs the generator 200 to adjust the microwave output stage 238 and/or the power supply 237 accordingly. Additionally, the controller 300 may also signal the supply pump to adjust the amount of cooling fluid to the microwave antenna 100 and/or the surrounding tissue. The controller 200 includes microprocessor 335 having memory 336 which may be volatile type memory (e.g., RAM) and/or non-volitile type memory (e.g., flash media, disk media, etc.). In the illustrated embodiment, the microprocessor 335 is in operative communication with the power supply 237 and/or microwave output stage 238 allowing the microprocessor 335 to control the output of the generator 300 according to either open and/or closed control loop schemes. The microprocessor 335 is capable of executing software instructions for processing data received by the temperature module 332, and for outputting control signals to the generator 300 and/or supply pump 40, accordingly. The software instructions, which are executable by the controller 300, are stored in the memory 336.

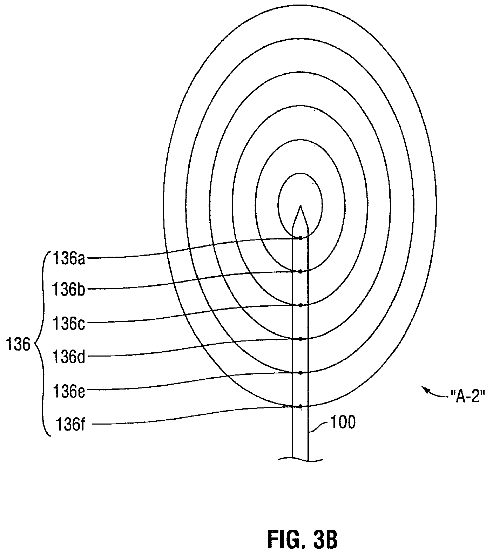

The microwave antenna 100 of the present disclosure is configured to create an ablation zone "A" having any suitable configuration, such as, for example, spherical (FIG. 3A), hemispherical, ellipsoidal (FIG. 3B where the ablation zone is designated "A-2"), and so forth. In one particular embodiment, microwave antenna 100 is configured to create an ablation zone "A" that is spherical (FIG. 3A). To facilitate understanding of the present disclosure, ablation zone "A" is being defined having a plurality of concentric ablation zones having radii $r_1$-$r_6$ when measured from the center of the ablation zone "A," collectively referred to as radii r. A measure of tissue temperature along any radius, e.g., $r_3$, associated with ablation zone "A" indicates tissue temperature at similar radii about the entire ablation zone "A." In one embodiment, a control algorithm of the present disclosure uses known (or in certain instances predicted) temperatures at specific radii to created an ablation zone "A" having a radius r. That is, temperatures associated with a specific radius are compiled into one or more look-up tables "D" and are stored in memory, e.g., memory 336, accessible by the microprocessor 335 and/or the temperature module 332. The temperatures associated with a specific radius and/or one or more look-up tables "D" may be stored into memory during the manufacture process of the generator 200, controller 300 or may be downloaded into memory 336 at a time prior to use of the system 10. When a temperature of the ablation zone "A" reaches a predetermined threshold temperature, the analog response produced by one of the corresponding thermal sensors 136, e.g., thermal sensor 136c, causes temperature module 332 to trigger a command signal to the controller 200 to adjust the power output accordingly. This combination of events will provide an ablation zone "A" with a radius approximately equal to $r_3$. Alternatively, or in combination therewith, the control algorithm, the microprocessor 335 and/or the temperature module 332 may utilize the above Arrhenius Equation to determine a specified amount of tissue damage, e.g., a threshold $\Omega(t)$, that correlates to ablation zone having a radius r. More particularly, for a given threshold $\Omega(t)$, a threshold temperature "T" may be determined and assigned a suitable voltage value that corresponds to a specific thermal sensor 136, thermal sensor 136c. In this instance, when a temperature of the ablation zone "A" reaches a predetermined threshold temperature, the analog response produced by one of the corresponding thermal sensors 136, e.g., thermal sensor 136c, causes temperature module 332 to trigger a command signal to the controller 200 to adjust the power output accordingly. This combination of events will provide an ablation zone "A" with a radius approximately equal to $r_3$. In embodiments, one or more control algorithms may utilize interpolation between the radii associated with the thermal sensors 136 to calculate temperatures between discreetly measured radii, e.g., temperatures measured between $r_3$ and $r_4$. More particularly, various (and commonly known) interpolation techniques may be utilized via curve fitting along the thermal sensors 136.

Temperature module 332 is in operative communication with the plurality of sensors 136 strategically located for sensing various properties or conditions, e.g., tissue temperature, antenna temperature, etc. Temperature module 332 may be a separate module from the microprocessor 335, or temperature module 332 may be included with the microprocessor 335. In an embodiment, the temperature module 332 may be operably disposed on the microwave antenna 100. The temperature module 332 may include control circuitry that receives information from the thermal sensors 136, and provides the information and the source of the information (e.g., the particular temperature sensor, e.g., 136c providing the information) to the controller 300 and/or microprocessor 335. In this instance, the thermal module 332, microprocessor 335 and/or controller 300 may access look-up table "D" and confirm that the threshold temperature at radius $r_3$ has been met and, subsequently instruct the generator 200 to adjust the amount of microwave energy being delivered to the microwave antenna. In one particular embodiment, look-up table "D" may be stored in a memory storage device (not shown) associated with the microwave antenna 100. More particularly, a look-up table "D" may be stored in a memory storage device operatively associated with handle 118 and/or connector 126 of the microwave antenna 100 and may be downloaded, read and stored into microprocessor 335 and/or memory 336 and, subsequently, accessed and utilized in a manner described above; this would do away with reprogramming the generator 200 and/or controller 300 for a specific microwave antenna. The memory storage device may also be configured to include information pertaining to the microwave antenna 100. Information, such as, for example, the type of microwave antenna, the type of tissue that the microwave antenna is configured to treat, the type of ablation zone desired, etc. may be stored into the storage device associated with the microwave antenna. In this instance, for example, generator 200 and/or controller 300 of system 10 may be adapted for use with a microwave antenna configured to create an ablation zone, e.g. ablation zone "A-2," different from that of microwave antenna 100 that is configured to create an ablation zone "A."

In the embodiment illustrated in FIG. 1A, the generator is shown operably coupled to fluid supply pump 40. The supply pump 40 is, in turn, operably coupled to the supply tank 44. In embodiments, the microprocessor 335 is in operative communication with the supply pump 40 via one or more suitable types of interfaces, e.g., a port 240 operatively disposed on the generator 200, which allows the microprocessor 335 to control the output of a cooling fluid 42 from the supply pump 40 to the microwave antenna 100 according to either open and/or closed control loop schemes. The controller 300 may signal the supply pump 40 to control the output of cooling fluid 42 from the supply tank 44 to the microwave antenna 100. In this way, cooling fluid 42 is automatically circulated to the microwave antenna 100 and back to the supply pump 40. In certain embodiments, a clinician may manually control the supply pump 40 to cause cooling fluid 42 to be expelled from the microwave antenna 100 into and/or proximate the surrounding tissue.

Operation of system 10 is now described. Initially, microwave antenna 100 is connected to generator 200. In one particular embodiment, one or more modules, e.g., AZCM 332, associated with the generator 200 and/or controller 300 reads and/or downloads data from a storage device associated with the antenna 100, e.g., the type of microwave antenna, the type of tissue that is to be treated, etc. Microwave antenna 100 including thermal sensors 136 may then be positioned adjacent tissue (FIG. 3A). Thereafter, the generator 200 may be activated supplying microwave energy to the radiating section 138 of the microwave antenna 100 such that the tissue may be ablated. During tissue ablation, when a predetermined threshold temperature is reached (such as the temperature that corresponds to radius $r_4$) a corresponding thermal sensor e.g., thermal sensor 136d, generates a voltage potential that is detected by the temperature module 332, which, in turn, instructs the generator 200 to adjust the microwave energy accordingly. In the foregoing sequence of events, the thermal sensor 136 and temperature module 332 function in real-time controlling the amount of microwave energy to the ablation zone such that a uniform ablation zone of suitable proportion is formed with minimal or no damage to adjacent tissue.

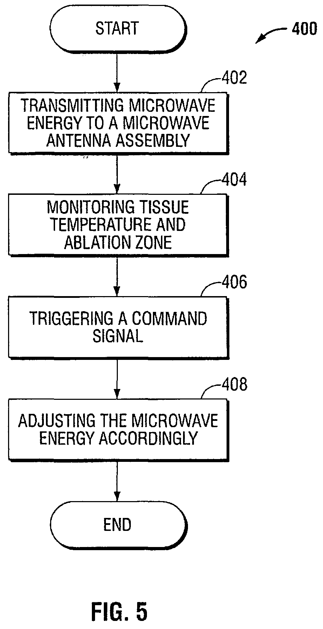

With reference to FIG. 5 a method 400 for monitoring temperature of tissue undergoing ablation is illustrated. At step 402, microwave energy from a generator 200 is transmitted to a microwave antenna 100 adjacent a tissue ablation site. At step, 404, tissue temperature at the ablation site is monitored. At step 406, a detection signal is triggered when a predetermined tissue temperature is reached at specific points along the microwave antenna. At step 408, the amount of microwave energy from the generator 200 to the microwave antenna may be adjusted.

From the foregoing and with reference to the various figure drawings, those skilled in the art will appreciate that certain modifications can also be made to the present disclosure without departing from the scope of the same. For example, in some embodiments, the disclosed methods may be extended to other tissue effects and energy-based modalities including, but not limited to, ultrasonic and laser tissue treatments. The method 400 is based on temperature measurement and monitoring, but other tissue and energy properties may be used to determine state of the tissue, such as current, voltage, power, energy, phase of voltage and current. In some embodiments, the method may be carried out using a feedback system incorporated into an electrosurgical system or may be a stand-alone modular embodiment (e.g., removable modular circuit configured to be electrically coupled to various components, such as a generator, of the electrosurgical system).

While system 10 has been described herein including individual thermal sensors 136 that correspond to a specific threshold tissue temperature, it is within the purview of the present disclosure that a single thermal sensor 136 may be configured to correspond to multiple threshold tissue temperatures that correspond to multiple radii. In this instance, the system 10 functions in substantially the same manner as described above.

While several embodiments of the disclosure have been shown in the drawings and/or discussed herein, it is not intended that the disclosure be limited thereto, as it is intended that the disclosure be as broad in scope as the art will allow and that the specification be read likewise. Therefore, the above description should not be construed as limiting, but merely as exemplifications of particular embodiments. Those skilled in the art will envision other modifications within the scope of the claims appended hereto.

**Claims**

1. A microwave antenna (100) adapted to connect to a power source (300) configured for performing an ablation procedure, comprising:

   - a radiating section (138) configured to deliver microwave energy from the power source (300) to tissue to form an ablation zone ("T"); and
   - a series of thermal sensors (136), the series of thermal sensors (136) being operably disposed adjacent the radiating section (138) of the microwave antenna (100), wherein the thermal sensors (136) extend proximally from the radiating section (138), the thermal sensors (136) corresponding to a radius of the ablation zone ("T"), wherein each thermal sensor (136) is configured to trigger a signal when a predetermined threshold tissue temperature is measured corresponding to the radius of the ablation zone, **characterized in that**
   the thermal sensors (136) are operably disposed within an internal generally circumferential wall (152) that surrounds and/or defines cooling fluid supply and fluid return lines (122,124).

2. A microwave antenna according to claim 1, wherein the series of thermal sensors are selected from the group consisting of thermistors, thermocouples, and fiber optical thermal monitoring devices.

3. A microwave antenna according to claim 1, wherein the series of thermal sensors (136) is arranged in a linear array along the shaft of the microwave antenna.

4. A microwave antenna according to claim 1, wherein the microwave antenna Is configured to produce an ablation zone that is spherical.

5. A system (10) for monitoring ablation size, comprising a power source (300) including a microprocessor (335) suitable for executing at least one control algorithm; and the microwave antenna (100) according to any of the preceding claims.

6. A system according to claim 5, further including a temperature module (332) configured to instruct the power source (300) to adjust the amount of microwave energy being delivered to the microwave antenna (100) when a signal from one of the series of thermal sensors (136) is received at the temperature module to create a uniform ablation zone ("A") of suitable proportion with minimal damage to adjacent tissue.

7. A system according to claim 6, wherein the at least one control algorithm calculates tissue necrosis for predicting the predetermined threshold temperature associated with the series of thermal sensors and corresponding radii.

8. A system according to claim 7, wherein the variables required to calculate tissue necrosis are selected from the group consisting of temperature, activation energy, gas constant, the rate constant, the pre-exponential factor and the gas constant

9. A system according to claim 7, further comprising:

   at least one fluid pump configured to supply a cooling fluid to the microwave antenna for facilitating cooling of one of the microwave antenna and tissue adjacent the ablation zone.

10. A system according to claim 6, wherein the temperature module is operably disposed within the power source.

11. A system according to claim 6, wherein the temperature module is operably disposed within the microwave antenna.

12. A system according to claim 6, wherein the temperature module (332) and series of thermal sensors (136) are configured to be activated when the power source (300) is activated.

13. A system according to any of claims 5 to 12, wherein the microprocessor (335) is configured to control the output of a cooling fluid (42) from a supply pump (40) to the microwave antenna (100) according to open and/or closed control loop schemes.

14. A system according to any of claims 5 to 12, wherein the microprocessor (335) is configured to control the output of a cooling fluid (42) from a supply pump (40) to the microwave antenna (100) according to a closed control loop scheme.

15. A system according to claim 6, wherein the temperature module (332) and series of thermal sensors (136) are configured to be activated when the power source (300) is deactivated.

## Patentansprüche

1. Mikrowellenantenne (100), die ausgebildet ist, an eine Stromquelle (300) angeschlossen zu werden, die konfiguriert ist, einen Ablationsvorgang ausführen, und die Folgendes umfasst:

   - einen Strahlungsteil (138), der konfiguriert ist, Mikrowellenenergie von der Stromquelle (300) an ein Gewebe zu liefern, um einen Ablationsbereich ("T") zu bilden, und
   - eine Reihe von thermischen Sensoren (136), wobei die Reihe der thermischen Sensoren (136) funktionsmäßig benachbart zum Strahlungsteil (138) der Mikrowellenantenne (100) angeordnet ist, wobei die thermischen Sensoren (136) sich proximal vom Strahlungsteil (138) erstrecken, wobei die thermischen Sensoren (136) einem Radius des Ablationsbereichs ("T") entsprechen, wobei jeder thermische Sensor (136) konfiguriert ist, ein Signal auszulösen, wenn eine vorbestimmte Schwellentemperatur des Gewebes entsprechend dem Radius des Ablationsbereichs gemessen wird,

   **dadurch gekennzeichnet, dass**
   die thermischen Sensoren (136) funktionsmäßig in einer inneren allgemein umgebenden Wand (152) angeordnet sind, die Kühlfluidzufuhr- und Fluidrücklaufleitungen (122, 124) umgibt und/oder diese definiert.

2. Mikrowellenantenne nach Anspruch 1, wobei die Reihe der thermischen Sensoren aus der Gruppe bestehend aus Thermistoren, Thermoelementen und faseroptischen thermischen Überwachungseinrichtungen ausgewählt ist.

3. Mikrowellenantenne nach Anspruch 1, wobei die Reihe der thermischen Sensoren (136) in einer linearen Anordnung entlang dem Schaft der Mikrowellenantenne angeordnet ist.

4. Mikrowellenantenne nach Anspruch 1, wobei die Mikrowellenantenne konfiguriert ist, einen Ablationsbereich zu erzeugen, der kugelförmig ist.

5. System (10) zur Überwachung der Ablationsgröße, das eine Stromquelle (300) mit einem Mikroprozessor (335), der geeignet ist, mindestens einen Steueralgorithmus auszuführen, und die Mikrowellenantenne (100) nach einem der vorhergehenden Ansprüche umfasst.

6. System nach Anspruch 5, welches ferner ein Temperaturmodul (332) umfasst, das konfiguriert ist, die Stromquelle (300) anzuweisen, die Menge der an die Mikrowellenantenne (100) gelieferten Mikrowellenenergie einzustellen, wenn ein Signal von einem aus der Reihe der thermischen Sensoren (136) am Temperaturmodul empfangen wird, um einen gleichförmigen Ablationsbereich ("A") mit einem geeigneten Maß und minimaler Beschädigung des benachbarten Gewebes zu bilden.

7. System nach Anspruch 6, wobei der mindestens eine Steueralgorithmus die Gewebenekrose berechnet, um die der Reihe der thermischen Sensoren und den entsprechenden Radien zugeordnete vorbestimmte Schwellentemperatur vorherzusagen.

8. System nach Anspruch 7, wobei die zum Berechnen der Gewebenekrose benötigten Variablen aus der Gruppe bestehend aus Temperatur, Aktivierungsenergie, Gaskonstante, Geschwindigkeitskonstante, dem präexponentiellen Faktor und der Gaskonstante ausgewählt sind.

9. System nach Anspruch 7, das ferner Folgendes umfasst:

   mindestens eine Fluidpumpe, die konfiguriert ist, ein Kühlfluid zur Mikrowellenantenne zum Erleichtern der Kühlung der Mikrowellenantenne oder des zum Ablationsbereich benachbarten Gewebes zuzuführen.

10. System nach Anspruch 6, wobei das Temperaturmodul funktionsmäßig in der Stromquelle angeordnet ist.

11. System nach Anspruch 6, wobei das Temperaturmodul funktionsmäßig in der Mikrowellenantenne angeordnet ist.

12. System nach Anspruch 6, wobei das Temperaturmodul (332) und die Reihe der thermischen Sensoren (136) konfiguriert sind, aktiviert zu werden, wenn die Stromquelle (300) aktiviert ist.

**13.** System nach einem der Ansprüche 5 bis 12, wobei der Mikroprozessor (335) konfiguriert ist, die Abgabe eines Kühlfluids (42) von einer Zufuhrpumpe (40) an die Mikrowellenantenne (100) gemäß einem offenen und/oder geschlossenen Regelkreis zu steuern.

**14.** System nach einem der Ansprüche 5 bis 12, wobei der Mikroprozessor (335) konfiguriert ist, die Abgabe eines Kühlfluids (42) von einer Zufuhrpumpe (40) an die Mikrowellenantenne (100) gemäß einem geschlossenen Regelkreis zu steuern.

**15.** System nach Anspruch 6, wobei das Temperaturmodul (332) und die Reihe der thermischen Sensoren (136) konfiguriert sind, aktiviert zu werden, wenn die Stromquelle (300) deaktiviert ist.

**Revendications**

**1.** Antenne à micro-ondes (100) qui est à même de se connecter à une source d'énergie (300) configurée pour effectuer une procédure d'ablation, comprenant :

- une section de rayonnement (138) configurée pour délivrer de l'énergie de micro-ondes de la source d'énergie (300) à un tissu afin de former une zone d'ablation (XT') ; et
- une série de capteurs thermiques (136), la série de capteurs thermiques (136) étant disposée en service adjacente à la section de rayonnement (138) de l'antenne à micro-ondes (100), dans laquelle les capteurs thermiques (136) s'étendent de manière proximale de la section de rayonnement (138), les capteurs thermiques (136) correspondant à un rayon de la zone d'ablation ('T'), dans laquelle chaque capteur thermique (136) est configuré pour déclencher un signal lorsqu'une température de tissu de seuil prédéterminée est mesurée, qui correspond au rayon de la zone d'ablation,

**caractérisée en ce qu** :

les capteurs thermiques (136) sont disposés en service dans une paroi interne généralement circonférentielle (152) qui entoure et/ou définit des conduites d'alimentation en fluide de refroidissement et de retour de fluide (122, 124).

**2.** Antenne à micro-ondes selon la revendication 1, dans laquelle la série de capteurs thermiques sont choisis dans le groupe constitué des thermistors, des thermocouples et des dispositifs de surveillance thermique à fibres optiques.

**3.** Antenne à micro-ondes selon la revendication 1, dans laquelle la série de capteurs thermiques (136) sont aménagés en réseau linéaire le long de l'arbre de l'antenne à micro-ondes.

**4.** Antenne à micro-ondes selon la revendication 1, dans laquelle l'antenne à micro-ondes est configurée pour produire une zone d'ablation qui est sphérique.

**5.** Système (10) pour surveiller la taille de l'ablation, comprenant une source d'énergie (300), comportant un microprocesseur (335) convenant pour exécuter au moins un algorithme de commande ; et l'antenne à micro-ondes (100) selon l'une quelconque des revendications précédentes.

**6.** Système selon la revendication 5, comprenant en outre un module de température (332) configuré pour instruire la source d'énergie (300) d'ajuster la quantité d'énergie de micro-ondes à délivrer à l'antenne à micro-ondes (100) lorsqu'un signal de l'un de la série de capteurs thermiques (136) est reçu par le module de température pour créer une zone d'ablation uniforme ('A) de proportion appropriée avec un dommage minimal au tissu adjacent.

**7.** Système selon la revendication 6, dans lequel le au moins un algorithme de commande calcule la nécrose du tissu pour prédire la température de seuil prédéterminée associée à la série de capteurs thermiques et de rayons correspondants.

**8.** Système selon la revendication 7, dans lequel les variables requises pour calculer la nécrose tissulaire sont choisies dans le groupe constitué de la température, de l'énergie d'activation, de la constante des gaz, de la constante de vitesse, du facteur pré-exponentiel et de la constante des gaz.

**9.** Système selon la revendication 7, comprenant en outre :

au moins une pompe à fluide configurée pour fournir un fluide de refroidissement à l'antenne à micro-ondes afin de faciliter le refroidissement de l'un(e) ou l'autre de l'antenne à micro-ondes et du tissu adjacent à la zone d'ablation.

**10.** Système selon la revendication 6, dans lequel le module de température est disposé en service dans la source d'énergie.

**11.** Système selon la revendication 6, dans lequel le module de température est disposé en service dans l'antenne à micro-ondes.

**12.** Système selon la revendication 6, dans lequel le module de température (332) et la série de capteurs thermiques (136) sont configurés pour être activés lorsque la source d'énergie (300) est activée.

13. Système selon l'une quelconque des revendications 5 à 12, dans lequel le microprocesseur (335) est configuré pour commander la délivrance d'un fluide de refroidissement (42) d'une pompe d'alimentation (40) à l'antenne à micro-ondes (100) selon des configurations à boucle de commande ouverte et/ou fermée.

14. Système selon l'une quelconque des revendications 5 à 12, dans lequel le microprocesseur (335) est configuré pour commander la délivrance d'un fluide de refroidissement (42) d'une pompe d'alimentation (40) à l'antenne à micro-ondes (100) selon une configuration à boucle de commande fermée.

15. Système selon la revendication 6, dans lequel le module de température (332) et la série de capteurs thermiques (136) sont configurés pour être activés lorsque la source d'énergie (300) est désactivée.

FIG. 1A

FIG. 1B

EP 2 361 583 B1

FIG. 2A

FIG. 2B

EP 2 361 583 B1

FIG. 3A

FIG. 3B

**FIG. 4**

FIG. 5

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6500175 B1
- US 6132425 A
- US 40126809 A